# EUROPEAN PATENT SPECIFICATION

(11) **EP 3 025 674 B1**
(45) Date of publication and mention of the grant of the patent: **01.08.2018**
(21) Application number: 14830236.7
(22) Date of filing: 24.06.2014
(51) Int. Cl.: A61B 34/30, A61B 34/00

(54) **THERAPEUTIC MANIPULATOR AND MANIPULATOR SYSTEM**
THERAPEUTISCHER MANIPULATOR UND MANIPULATORSYSTEM
MANIPULATEUR THÉRAPEUTIQUE ET SYSTÈME DE MANIPULATEUR

(30) Priority: 26.07.2013 JP 2013155883
(43) Date of publication of application: 01.06.2016
(73) Proprietor: Olympus Corporation, Tokyo 192-8507 (JP)
(72) Inventor: YANAGIHARA, Masaru, Tokyo 192-8507 (JP); KISHI, Kosuke, Tokyo 192-8507 (JP)
(74) Representative: Schicker, Silvia
(86) International application number: PCT/JP2014/066634
(87) International publication number: WO 2015/012044

(56) References cited:
- WO-A2-2006/124390
- WO-A2-2007/146987
- CA-A1- 2 437 286
- JP-A- 2003 204 968
- JP-A- 2004 122 286
- JP-A- 2009 539 573
- JP-A- 2012 055 996
- US-A- 5 813 976
- US-A1- 2010 331 856
- US-A1- 2010 331 856

## Description

### {Technical Field}

The present invention relates to a treatment manipulator and a manipulator system.

### {Background Art}

In the related art, there are known treatment manipulators having two arms provided with two bending portions that are adjacent to each other in the longitudinal direction at the distal end of an inserted portion (for example, see JP2010057914). In addition, there are known treatment manipulators provided with, in order to perform treatment in a lateral direction with respect to an inserted portion, an arm having two flexing joints that are adjacent to each other in the longitudinal direction and a roll joint disposed on the base-end side thereof (for example, see US8057385). WO2007/146987 A2 refers to a minimally invasive surgical system including a surgical instrument extending through a guide tube, the surgical instrument having a distal end movable, by means of actuators that are telemanipulatively controlled, in all six Cartesian degrees of freedom independently of other components of a telemanipulated surgical system. On the other hand, WO2006/124390 A2 discloses telerobotic, telesurgical, and/or surgical robotic devices and systems which include surgical robotic linkages that may have more degrees of freedom than an associated surgical end effector. A processor calculates a tool motion that includes pivoting of the tool about an aperture site. Linkages movable along a range of configurations for a given end effector position may be driven toward configurations which inhibit collisions.
Further prior art related to a treatment manipulator includes the following Patent Literature: CA 2 437 286 A1 and US 2010/331856 A1.

### {Citation List}

### {Patent Literature}

{PTL 1} Japanese Unexamined Patent Application, Publication No. 2010-57914
{PTL 2} Publication of U.S. Patent No. 8057385 Specification
{PTL 3} International Patent Application Publication No. 2007/146987 A2
{PTL 4} International Patent Application Publication No. 2006/124390 A2

### {Summary of Invention}

### {Technical Problem}

However, with the treatment manipulator of JP2010057914, because the joints at the distal-end side for changing the orientation of a treatment tool have gently curved shapes, there is a problem in that it is not possible to treat an affected portion located in the vicinity of the inserted-portion main unit. In addition, with the treatment manipulator of US8057385, because the roll joint at the base must be operated in order to move the treatment tool at the distal end in a direction that intersects the direction of movement caused by the flexing joint, nearly the entire treatment manipulator ends up moving, which results in the problem of the treatment tool coming into contact with tissue or the like in the surrounding area thereof.

The present invention has been conceived in light of the above-described circumstances, and an object thereof is to provide a treatment manipulator and a manipulator system with which it is possible to treat an affected portion located at a position in the close vicinity of an inserted-portion main unit while reducing interference with tissue or the like in a surrounding area.

### {Solution to Problem}

In order to achieve the above-described object, the present invention provides the following solution according to claim 1. Preferred embodiments are found in the dependent claims. An aspect of the present disclosure provides a treatment manipulator including the features of claim 1. A treatment manipulator may include an inserted-portion main unit; at least an arm that is provided so as to protrude forward from a distal-end surface of the inserted-portion main unit and that has an end effector at a distal end thereof; and an endoscope that is provided in the inserted-portion main unit and that has a viewing-field area in which the end effector at the distal end of the arm can be observed, wherein the arm is provided with, sequentially from the distal-end side, a first flexing joint that can pivot the end effector about a first axis orthogonal to a longitudinal axis of the arm, an intermediate roll joint that can be rotated about the longitudinal axis, and a second flexing joint that can be pivoted about a second axis orthogonal to the longitudinal axis, and the first flexing joint can be flexed 90° or more at least on one side with respect to the longitudinal axis.

With this aspect, when the inserted-portion main unit is inserted into the body cavity from the distal-end-surface side, the arm is disposed so as to protrude forward from the distal-end surface, and thus, an inner wall of the body cavity in front of the inserted portion can be treated by using the arm. In this case, by using the first flexing joint disposed on the distal-end side, which can be flexed 90° or more, treatment can be applied by using the end effector at the distal end by easily accessing the site located at a position in the close vicinity of the distal-end surface of the apparatus main unit.

In this case, the intermediate roll joint is rotated in order to move the end effector in a direction that intersects with the plane of flexing performed by the first flexing joint, when the end effector is treating the site in the close vicinity of the apparatus main unit in the state in which the first flexing joint is flexed in the body cavity. When the intermediate roll joint disposed between the first flexing joint and the second flexing joint is rotated, it is possible to rotate only the first flexing joint and the end effector disposed closer to the distal-end side than the intermediate roll joint is, and thus, it is possible to reduce interference between tissue in the surrounding area and the arm by decreasing the rotation radius.

In the above-described aspect, the inserted-portion main unit may be provided with a through-hole that passes through the inserted-portion main unit in a longitudinal direction so as to open at the distal-end surface thereof and that accommodates the endoscope in a movable manner, and the through-hole may be provided with a notch having a larger width than an outer diameter of the endoscope over a predetermined length from an opening at the distal-end surface.

By doing so, the inserted-portion main unit is inserted into the body cavity in the state in which the endoscope is accommodated in the through-hole provided in the inserted-portion main unit, the endoscope accommodated in the through-hole is moved in the longitudinal direction of the through-hole in the close vicinity of a site to be treated, and thus, the endoscope can be moved forward/backward in the longitudinal direction so as to be protruded from the opening at the distal-end surface of the inserted-portion main unit. With the protruded endoscope, it is possible to observe the end effector disposed at the distal end of the arm by directing the field of view in a desired direction by bending a bending portion provided in the endoscope.

In this case, when the endoscope is retracted in the longitudinal direction of the through-hole while the endoscope that is made to protrude from the opening at the distal-end surface of the inserted-portion main unit is kept in the curved form, by accommodating an intermediate position of the endoscope in the length direction thereof in the notch provided at the distal end of the through-hole, it is possible to make the endoscope in the through-hole protrude radially outward from the inserted-portion main unit via the notch. Therefore, it is possible to move the endoscope forward/backward while keeping the bending portion bent, and thus, it is possible to observe the same site while moving the viewing-field area forward/backward and while changing angles.

In addition, in the above-described aspect, a driving wire rod that drives the end effector may be disposed so as to pass through interiors of the individual joints constituting the arm.

By doing so, it is possible to drive the end effector while preventing the driving wire rod from sticking out from the arm, and thus, it is possible to prevent the problem of the driving wire rod interfering with the field of view.

In addition, in the above-described aspect of the invention, the first flexing joint is allowed to be flexed 90° or more only on one side with respect to the longitudinal axis, and the first flexing joint has, at the interior thereof, a pathway through which the driving wire rod passes, and is provided with a pivoting member that is passively pivoted about a flexing center of the first flexing joint in response to the operation of the bending driving wire rod.

By doing so, it is possible to minimize the slackness of the driving wire rod disposed in the pathway inside the first flexing joint, and, by passively pivoting the pivoting member in response to the movement of the driving wire rod, it is possible to prevent the driving wire rod from being subjected to an excessive load. By doing so, even if the first flexing joint is pivoted between the state in which the fist flexing joint is extended in the direction along the longitudinal axis and the state in which the first flexing joint is flexed 90° or more on one side, it is possible to prevent the driving wire rod from protruding radially outward from the arm due to the slackness thereof and forming a kink by being subjected to an excessive load.

In addition, the above-described aspect may be provided with a base-end-side roll joint that is provided closer to the base-end side than the second flexing joint is so as to be rotatable about the longitudinal axis.

By doing so, when the base-end-side roll joint is rotated, it is possible to rotate nearly the entire arm including the portion from the end effector to the second flexing joint. Accordingly, it is possible to withdraw the arm from the field of view of the endoscope by rotating the base-end-side roll joint within an area in which the arm and the inner wall of the body cavity do not interfere with each other.

In addition, the above-described aspect may be provided with a guide groove that is provided in an outer surface of the inserted-portion main unit along the longitudinal direction thereof, that has an opening part that opens radially outward, and into which a tubular guide member can be inserted from a radially outward direction via the opening part.

By doing so, in the state in which the tubular guide member in introduced inside the body cavity, the inserted-portion main unit is mounted to the guide member so that the portion of the guide member disposed outside the body is accommodated in the guide groove from the opening part, the inserted-portion main unit is moved along the longitudinal direction of the guide member, and thus, it is possible to easily insert the inserted-portion main unit into the body cavity. The guide member is, for example, an endoscope.

In addition, another aspect of the present invention provides a manipulator system including a slave apparatus provided with any one of the above-described treatment manipulators and a driving portion that drives the treatment manipulator; a master apparatus provided with a manipulation portion that is manipulated by an operator; and a controller that controls the driving portion of the slave apparatus based on an input signal input via the manipulation portion of the master apparatus.

In the above-described aspect, when generating operation instruction signals for the individual joints of the slave apparatus based on input signals received from the master apparatus, the controller may calculate the operation instruction signals for the individual joints so that the first flexing joint is disposed at a position further away from a center of an endoscope image.

### {Advantageous Effects of Invention}

The present invention affords an advantage in that it is possible to treat an affected portion located at a position in the close vicinity of an inserted-portion main unit while reducing interference with tissue or the like in a surrounding area.

### {Brief Description of Drawings}

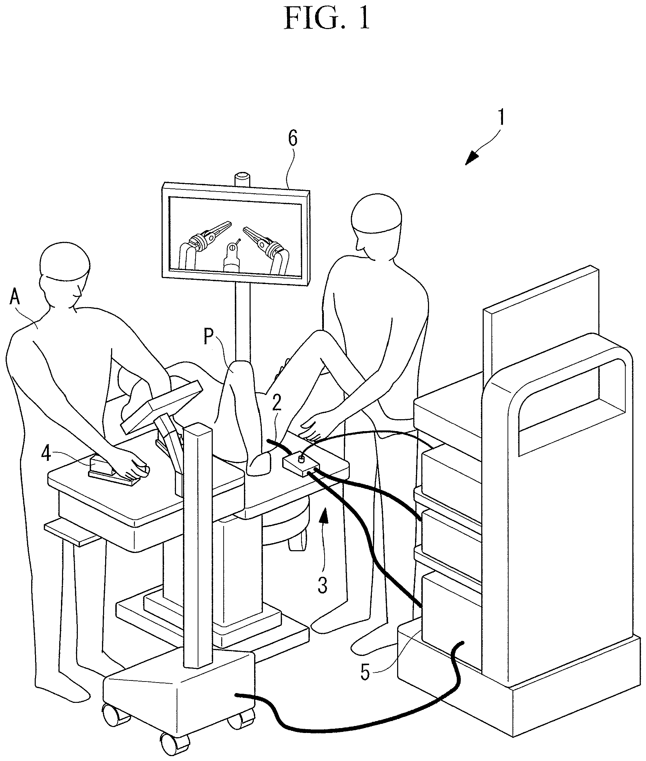

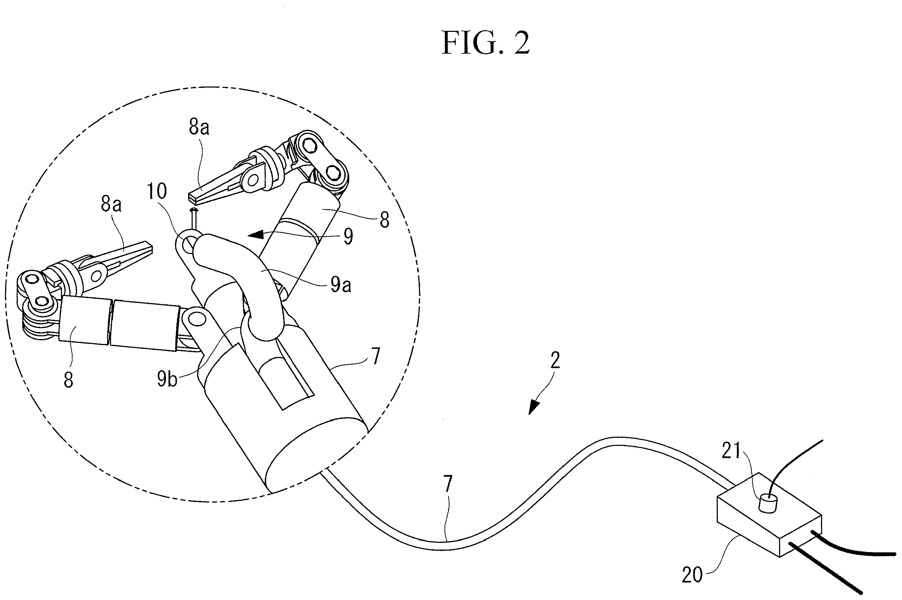

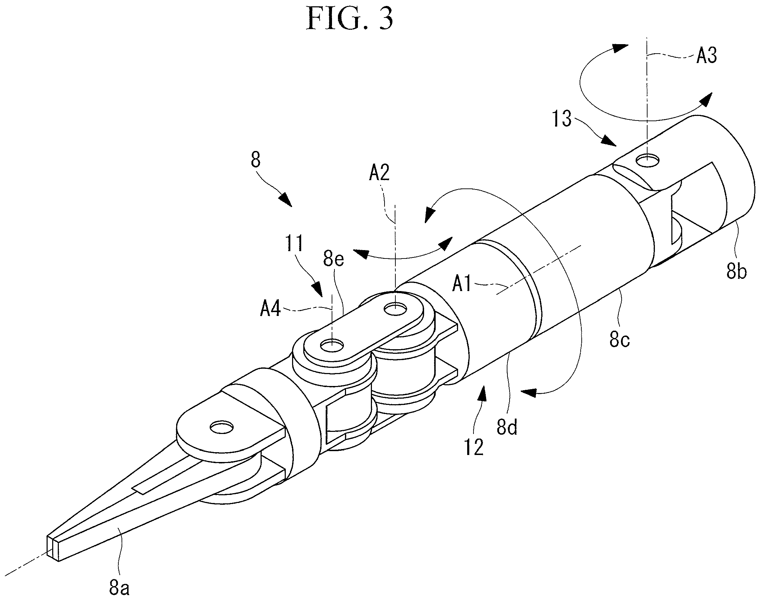

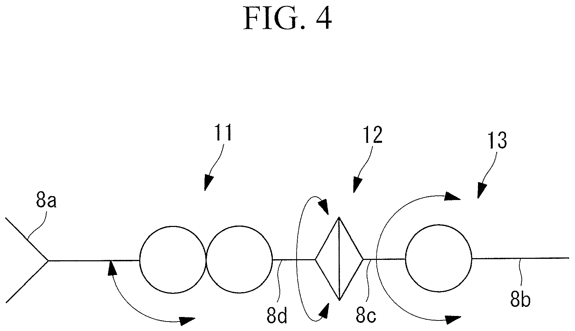

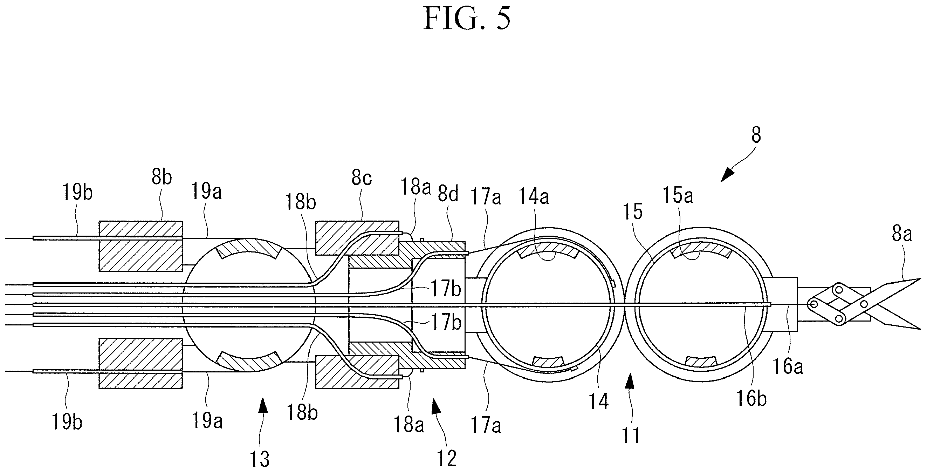

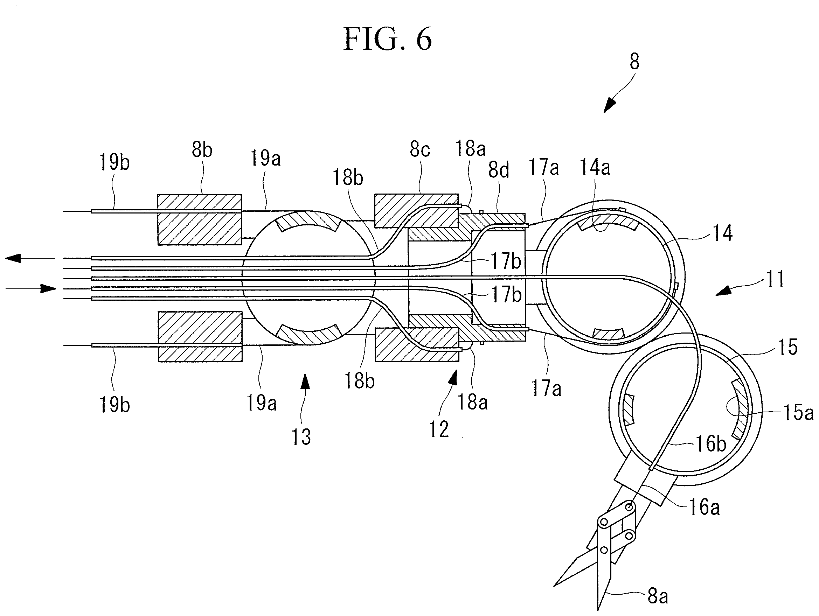

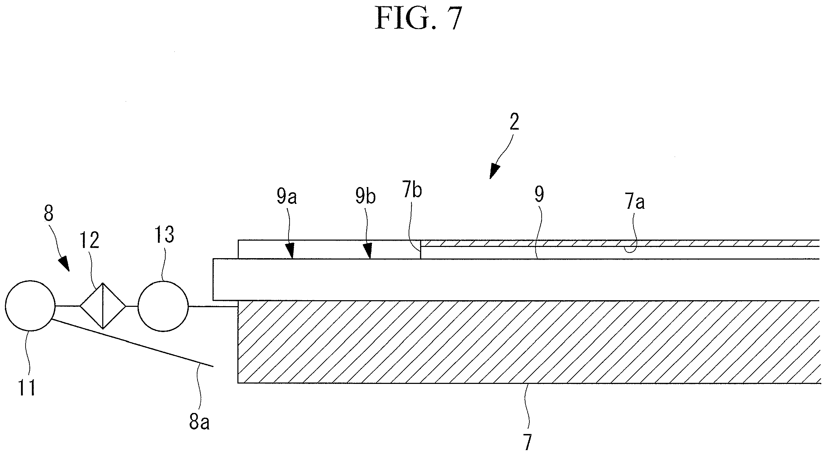

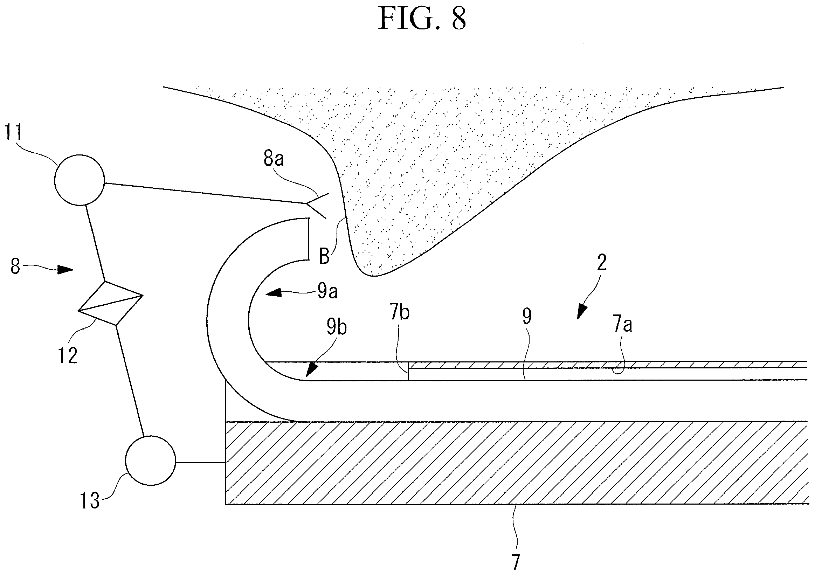

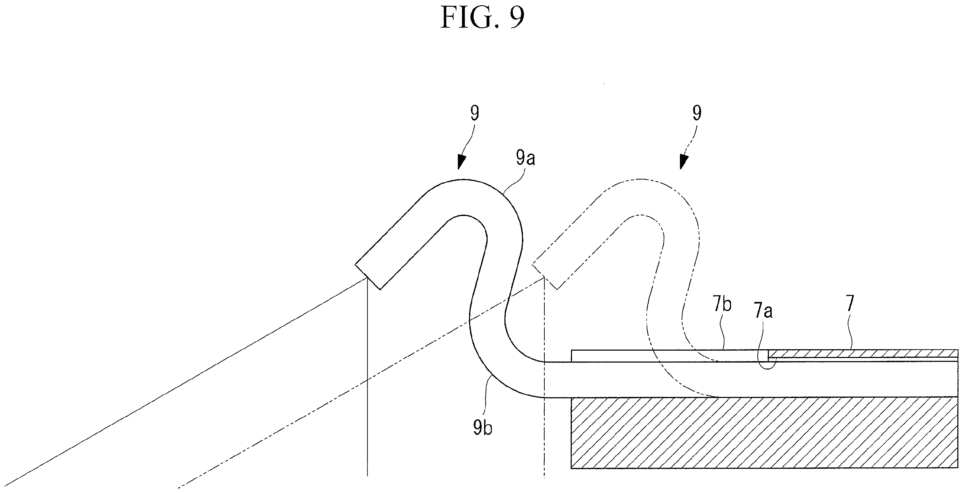

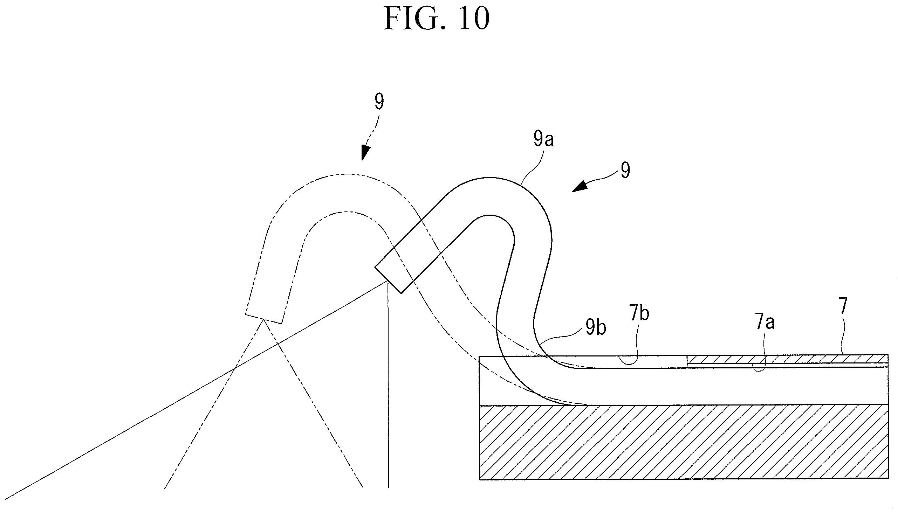

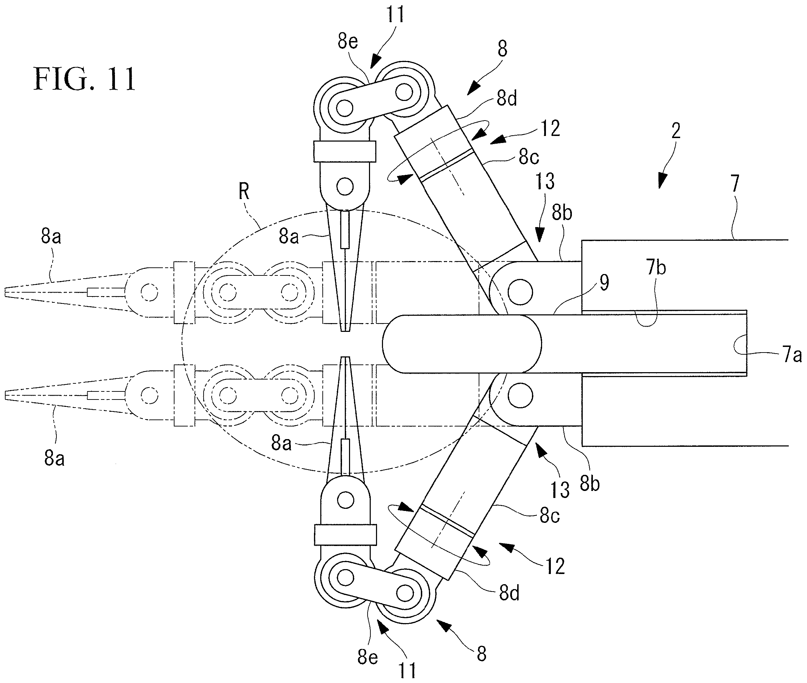

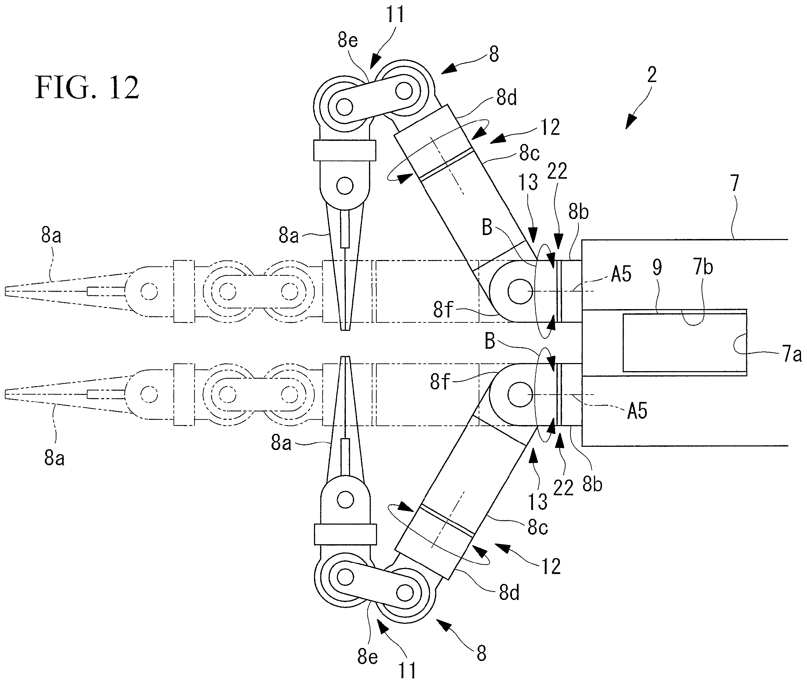

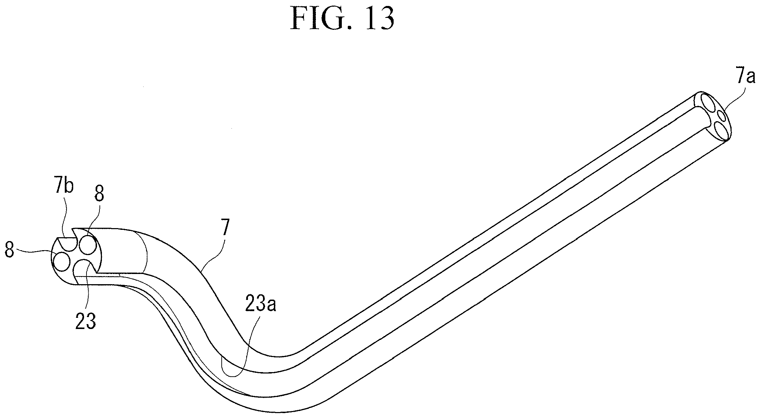

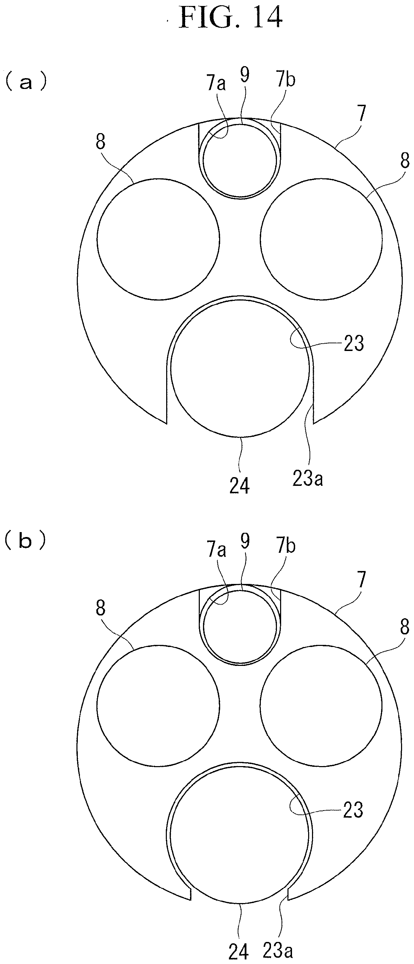

{Fig. 1} Fig. 1 is an overall configuration diagram showing a manipulator system provided with a treatment manipulator according to an embodiment of the present invention.
{Fig. 2} Fig. 2 is a perspective view showing the treatment manipulator according to the embodiment of the present invention provided in the manipulator system in Fig. 1.
{Fig. 3} Fig. 3 is a perspective view showing an arm provided in the treatment manipulator in Fig. 2.
{Fig. 4} Fig. 4 is a schematic diagram showing the shaft configuration of the arm in Fig. 3.
{Fig. 5} Fig. 5 is a schematic diagram showing an internal structure in a state in which the arm in Fig. 3 is extended in a straight line.
{Fig. 6} Fig. 6 is a schematic diagram showing the internal structure in a state in which a first flexing joint of the arm in Fig. 3 is bent.
{Fig. 7} Fig. 7 is a schematic diagram for explaining a form of the treatment manipulator in Fig. 2 when inserted into the body cavity.
{Fig. 8} Fig. 8 is a schematic diagram for explaining a form for treating an affected portion located in an opposite direction from the direction of insertion by using the treatment manipulator in Fig. 2.
{Fig. 9} Fig. 9 is a partial sectional view showing an operation example of an endoscope provided in the treatment manipulator in Fig. 2.
{Fig. 10} Fig. 10 is a partial sectional view showing another operation example of the endoscope provided in the treatment manipulator in Fig. 2.
{Fig. 11} Fig. 11 is a partial front view of a distal-end portion for explaining the operation of the treatment manipulator in Fig. 2.
{Fig. 12} Fig. 12 is a partial front view showing a first modification of the treatment manipulator in Fig. 2.
{Fig. 13} Fig. 13 is a perspective view showing an inserted-portion main unit provided in the treatment manipulator in Fig. 2.
{Fig. 14} Fig. 14 shows (a) a front view of the inserted-portion main unit in Fig. 13 and (b) a front view of a modification thereof.

### {Description of Embodiment}

A treatment manipulator and a manipulator system according to an embodiment will be described below with reference to the drawings.

As shown in Fig. 1, a manipulator system 1 according to this embodiment is provided with a slave apparatus 3 including a treatment manipulator 2 according to this embodiment that is inserted into a body cavity of a patient P and a driving portion (not shown) thereof; a master apparatus 4 including a manipulation portion that is manipulated by an operator A; a controller 5 that controls the driving portion of the slave apparatus 3 based on input signals generated by manipulating the master apparatus 4; and a monitor 6 that displays an image acquired by the treatment manipulator 2.

As shown in Fig. 2, the treatment manipulator 2 according to this embodiment is provided with a flexible tubular inserted-portion main unit 7, two arms 8 that are provided so as to protrude forward from a distal-end surface of the inserted-portion main unit 7, and an endoscope 9 that is accommodated, in a movable manner, inside a through-hole 7a (see Fig. 7) provided along the longitudinal direction of the inserted-portion main unit 7. In the figure, the broken line indicates an enlarged view of a distal-end portion of the inserted-portion main unit 7, and reference sign 10 is a treatment tool that is introduced via a forceps channel 7b (see Fig. 7) that is provided along the longitudinal direction of the inserted-portion main unit 7 so as to be placed in a manner protruding from the distal-end surface.

As shown in Figs. 3 and 4, the two arms 8 are each provided with, sequentially from the distal-end side along the longitudinal axial direction, an end effector 8a, such as grasping forceps, a first flexing joint 11 that can be flexed about an axis A2 that is orthogonal to a longitudinal axis A1, an intermediate roll joint 12 that can be rotated about the longitudinal axis A1, and a second flexing joint 13 that can be flexed about an axis A3 that is orthogonal to the longitudinal axis A1.

Although this embodiment will be described assuming that the arms 8 are secured to the inserted-portion main unit 7, the arms 8 may protrude from channels (not shown) in the inserted-portion main unit 7. Even if the arms 8 protrude from the channels, the arms 8 are operated similarly to the case in which the arms 8 are secured to the inserted-portion main unit 7.

The second flexing joint 13 supports a columnar first arm portion 8c on a columnar secured portion 8b that is secured to the inserted-portion main unit 7, so as to be pivotable over a ±90° angular range with respect to the longitudinal axis of the secured portion 8b about the axis A3 that is orthogonal to the longitudinal axis of the secured portion 8b.

The intermediate roll joint 12 supports a columnar second arm portion 8d coaxially arranged with the first arm portion 8c so as to be rotatable by ±180° with respect to the first arm portion 8c about the longitudinal axis A1 thereof.

The first flexing joint 11 couples the second arm portion 8d and the end effector 8a via a linkage member 8e, and is a joint employing a double-joint system in which the linkage member 8e is pivoted with respect to the second arm portion 8d, and the end effector 8a is pivoted with respect to the linkage member 8e, about axes A2 and A4, respectively, which are parallel to each other. Here, the joint employing the double-joint system is a joint that is provided with, for example, gears (not shown) that engage with each other in the second arm portion 8d and the end effector 8a, and with which, when the linkage member 8e is pivoted about the axis A2, the end effector 8a is rotated about the axis A4, thus also being pivoted with respect to the linkage member 8e.

With the first flexing joint 11 employing the double-joint system, the end effector 8a can be pivoted by a larger angle than the pivoting angle about the axis A2, and thus, it is possible to realize an operation over a large angular range. By doing so, the first flexing joint 11 is configured so that the effector 8a can be flexed over a 170° angular range only on one side of the longitudinal axis A1 of the second arm portion 8d.

Note that the first flexing joint 11 is not limited to the above-described joint employing the double-joint system; specifically, a joint that does not employ the linkage member 8e and that is rotatable about a single rotation axis may be employed.

As shown in Figs. 5 and 6, the first flexing joint 11 is provided with discoid pivoting members 14 and 15 that are supported so as to be pivotable about the two axes A2 and A4, respectively. The pivoting members 14 and 15 are provided with pathways 14a and 15a, respectively, which radially pass through the interiors thereof. The pathways 14a and 15a have large enough heights in the directions of the axes A2 and A4 to accommodate a sheath 16b through which a drive wire 16a that drives the end effector 8a passes and widths that are sufficiently larger than the sheath 16b in the direction orthogonal to the axes A2 and A4. Note that, without limitation to the drive wire 16a, a long, thin rod possessing flexibility may be employed.

In Figs. 5 and 6, reference sign 17a is a wire for driving the first flexing joint 11, reference sign 17b is a sheath through which the wire 17a passes, reference sign 18a is a wire for driving the intermediate roll joint 12, reference sign 18b is a sheath through which the wire 18a passes, reference sign 19a is a wire for driving the second flexing joint 13, and reference sign 19b is sheath through which the wire 19a passes.

The endoscope 9 is provided with an observation optical system (not shown) having a viewing-field area in front of the distal-end surface thereof, also has two bending portions 9a and 9b that are adjacent to each other with a space therebetween in the longitudinal direction, and is configured so that it is possible to take a straight form in which the bending portions 9a and 9b are straightened, as shown in Fig. 7, and to take an S-shaped form in which the two bending portions 9a and 9b are bent in opposite directions from each other, as shown in Fig. 2. Because the endoscope 9 can be accommodated inside the through-hole 7a of the inserted-portion main unit 7 by taking the straight form, it is possible to smoothly insert the endoscope 9 into the body cavity. On the other hand, by taking the S-shaped form, it is possible to direct the viewing-field area to a position close to the distal-end surface of the inserted-portion main unit 7.

In addition, as shown in Fig. 8, by bending the bending portions 9a and 9b of the endoscope 9 by 90° in the same direction, it is possible to ensure a satisfactory viewing field and perform treatment even for an affected portion and a body-cavity inner side surface B located in the opposite direction from the direction in which the inserted-portion main unit 7 is inserted.

In addition, in this embodiment, the inserted-portion main unit 7 is provided with, at the distal end thereof, a notch 7b that is formed by cutting out the through-hole 7a radially outward over a predetermined length from the distal-end surface in the longitudinal direction. The through-hole 7a is provided at an eccentric position with respect to the center axis of the inserted-portion main unit 7, and the notch 7b is formed facing the opposite side from the center axis of the inserted-portion main unit 7 with the through-hole 7a interposed therebetween. The width of the notch 7b is formed so as to be larger than the outer diameter of the endoscope 9.

By doing so, not to mention that the endoscope 9 can be accommodated inside the through-hole 7a in the state in which the endoscope 9 is straightened out in a straight line, as shown in Fig. 7, even in a state in which the bending portions 9a and 9b at the distal end are bent, so long as the endoscope 9 is bent to the notch 7b side, the endoscope 9 can be made to protrude radially outward from the inserted-portion main unit 7 via the notch 7b, as shown by the chain lines in Fig. 9.

Specifically, by moving the endoscope 9 forward/ backward in the longitudinal direction of the through-hole 7a in the state in which the bending portions 9a and 9b of the endoscope 9 are bent and fixed in an S-shape, as shown in Fig. 9, it is possible to translationally move the field of view located in the vicinity of the distal-end surface of the inserted-portion main unit 7 in the longitudinal direction of the inserted-portion main unit 7. In addition, as shown in Fig. 10, by changing the bending angles of the two bending portions 9a and 9b while moving the endoscope 9 forward/backward in the longitudinal direction of the through-hole 7a, it is possible to change the viewpoint for the same site.

As shown in Figs. 5 and 6, the first flexing joint 11, the intermediate roll joint 12, and the second flexing joint 13 are driven by the wires 17a, 18a, and 19a, respectively. The individual wires 17a, 18a, and 19a extend to the base-end side of the inserted-portion main unit 7 via the interior of the inserted-portion main unit 7. Then, the wires 17a, 18a, and 19a are connected to the driving portion disposed in the controller 5 via a relay portion 20 that is attached to the base-end side of the inserted-portion main unit 7 so as to be pushed and pulled in the longitudinal direction of the inserted-portion main unit 7.

In addition, as shown in Fig. 2, the relay portion 20 is provided with a treatment-tool port 21 so that the treatment tool 10 to be made to protrude from the distal-end surface of the inserted-portion main unit 7 is inserted into the forceps channel 7b of the inserted-portion main unit 7 via the treatment-tool port 21.

In addition, the endoscope 9 is provided with a manipulation portion (not shown) at the base-end portion that protrudes from the base-end side of the inserted-portion main unit 7 so that the bending portions 9a and 9b can be bent in arbitrary directions by manipulating the manipulation portion.

The operation of the thus-configured treatment manipulator 2 and manipulator system 1 according to this embodiment will now be described below.

As shown in Fig. 7, in order to perform treatment in the body cavity of the patient P by using the manipulator system 1 according to this embodiment, the flexing joints 11 of the two arms 8 that protrude from the distal-end surface of the inserted-portion main unit 7 of the treatment manipulator 2 are flexed closed to 180°, the intermediate roll joints 12 are rotated to an angle that allows the arms 8 to be accommodated inside the outer diameter of the inserted-portion main unit 7, the second flexing joints 13 are straightened out, and the inserted-portion main unit 7 is inserted into the body cavity, for example, the anus, of the patient P in the state in which the bending portions 9a and 9b of the endoscope 9 are straightened out.

Then, once the distal end of the inserted-portion main unit 7 is positioned in the vicinity of the affected portion, the operator A manipulates the master apparatus 4 to actuate the two arms 8 and also manipulates the manipulation portion of the endoscope 9 to place the viewing field at a position where the affected portion and the end effectors 8a at the distal ends of the arms 8 can be observed. An image captured by the endoscope 9 is displayed on the monitor 6, and the operator A manipulates the master apparatus 4 and the manipulation portion of the endoscope 9 while checking the monitor 6.

Because the two arms 8 each have two flexing joints 11 and 13 with a space therebetween in the longitudinal direction, by flexing the two flexing joints 11 and 13 in the opposite directions from each other, the end effectors 8a can be moved forward/backward like SCARAs (Selective Compliance Assembly Robot Arms), as shown in Fig. 11.

At this time, because the first flexing joints have a pivoting angle range of 170° on one side thereof, by flexing the end effectors so as to completely fold back toward the arms, it is possible to bring the distal ends of the end effectors close to the vicinity of the distal-end surface of the inserted-portion main unit.

Also, in this case, by flexing the first flexing joints 11 and the second flexing joints 13 of the two arms 8 in the opposite directions from each other, respectively, as indicated by the solid lines in Fig. 11, it is possible to place the end effectors 8a within a viewing-field area R of the endoscope 9, thus placing the arms 8 outside the viewing-field area R, which makes it possible to prevent the arms 8 from interfering with the viewing field.

Furthermore, by rotating the intermediate roll joints 12, the distal ends of the end effectors 8a that have been placed in the vicinity of the distal-end surface of the inserted-portion main unit 7 in this way can be moved in the direction (a direction that intersects the plane of Fig. 11) that intersects the plane (the plane parallel to the plane of Fig. 11) in which the end effectors 8a are operated by the first flexing joints 11.

In this case, because the intermediate roll joints 12 are disposed between the first flexing joints 11 and the second flexing joints 13, the portions of the arms 8 that are operated by rotating the intermediate roll joints 12 can be shorter as compared with the case in the related art in which the roll joints are placed closer to the base-end side than the second flexing joints 13 are, and thus, there is an advantage in that it is possible to reduce interference with tissue in the surrounding area by decreasing the rotation radius.

In addition, because the wires 16a for driving the end effectors 8a are inserted into the sheaths 16b that pass through the pathways 14a and 15a inside the pivoting members 14 and 15 disposed so as to be pivotable about the two axes A2 and A4 of the first flexing joints 11, the slackness of the sheaths 16b inside the pathways 14a and 15a changes between the state in which the first flexing joints 11 are flexed 170° and the state in which the first flexing joints 11 are straightened out. Because the pathways 14a and 15a inside the pivoting members 14 and 15 have sufficiently large widths, it is possible to accommodate the sheaths 16b in both cases.

Therefore, it is possible to prevent the wires 16a from protruding outside the arms 8 and thus interfering with the field of view. In addition, when tensile forces are exerted on the wires 16a, it is possible to prevent the wires 16a from being subjected to excessive forces by passively pivoting the pivoting members 14 and 15 by using the forces received from the wires 16a.

In addition, with the treatment manipulator 2 according to this embodiment, because the notch 7b is provided over the predetermined length from the distal-end surface of the inserted-portion main unit 7, it is possible to translationally move the viewing-field area R by moving the endoscope 9 in the longitudinal direction of the through-hole 7a while the endoscope 9 observing the affected portion by bending the bending portions 9a and 9b is kept in the bent state. This is particularly effective when observing an affected portion located at a position in the close vicinity of the distal-end surface of the inserted-portion main unit 7.

Note that, although the arms 8 each having the two flexing joints 11 and 13 and the intermediate roll joint 12 disposed therebetween have been described as examples in this embodiment, base-end-side roll joints 22 that rotate linkages 8f about axes A5 with respect to the secured portion 8b may be provided farther toward the base-end side than the second flexing joints 13 are, as shown in Fig. 12. By rotating the base-end-side roll joints 22, as indicated by the arrows B in Fig. 12, the flexed first flexing joints 11 can be rotated about the axes A5 of the secured portions 8b.

In the description according to Fig. 11, although the first flexing joints 11 and the second flexing joints 13 of the two arms 8 are flexed in the opposite directions from each other, and thus, the first flexing joints 11 are disposed so that both elbows are stuck out, by rotating the base-end-side roll joints 22, the first flexing joints 11 can be folded in a compact manner by placing the elbows in vertical positions. As a result, it is possible to decrease the contact between the arms 8 and the body-cavity inner walls in the area surrounding the arms 8 by bringing the first flexing joints 11 close to each other to an extent that the arms 8 do not interfere with the viewing field.

In addition, although the treatment tool 10 is assumed to be introduced via the forceps channel 7b formed of the through-hole provided in the inserted-portion main unit 7 in this embodiment, alternatively, a guide groove 23 having an opening part 23a provided at an outer circumferential surface of the inserted-portion main unit 7 may be provided over the entire length of the inserted-portion main unit 7, as shown in Fig. 13. The width of the opening part 23a of the guide groove 23 is a width that allows an observation endoscope (guide member) 24 (see Fig. 14) to be inserted thereinto.

By doing so, before inserting the treatment manipulator 2 into the body cavity, the observation endoscope 24 is introduced into the body cavity first, a portion of the observation endoscope 24 disposed outside the body of the patient P is accommodated in the guide groove 23 from the opening part 23a, while observing the interior of the body cavity, in the state in which the distal end of the observation endoscope 24 is placed in the vicinity of an affected portion, and thus, it is possible to more easily introduce the treatment manipulator 2 into the body cavity of the patient P by using the observation endoscope 24 as a guide.

In this case, by providing the guide groove 23 having the opening part 23a at the outer circumferential surface of the inserted-portion main unit 7, it is possible to mount the inserted-portion main unit 7 at an intermediate position, in the longitudinal direction, of a portion of the observation endoscope 24 that is exposed outside the body when the distal-end portion thereof is disposed inside the body cavity in the inserted state, and thus, there is an advantage in that it is possible to insert the treatment manipulator 2 into the body cavity without removing the observation endoscope 24 from the body cavity.

The guide groove 23 may be provided so as to open at the maximum width, as shown in Fig. 14(a), or the width of the opening part 23a may be smaller than the maximum width, as shown in Fig. 14(b). By accommodating the observation endoscope 24 in the guide groove 23 by pushing open the opening part 23a of the inserted-portion main unit 7 formed of a flexible material, it is possible to prevent the treatment manipulator 2 from becoming detached from the endoscope 24 while inserting the treatment manipulator 2 by using the endoscope 24 as a guide.

In addition, although the flexing angle of the first flexing joints 11 is assumed to be 170° on one side in this embodiment, it is not limited thereto, and the flexing angle of 90° or greater on one side is acceptable. In addition, although the angular range of the second flexing joints 13 is assumed to be ±90°, alternatively, it suffices that the second flexing joints 13 be bendable in an arbitrary angular range.

### {Reference Signs List}

A operator
A1 longitudinal axis
A2 first axis
A3 second axis
R viewing-field area
1 manipulator system
2 treatment manipulator
3 slave apparatus
4 master apparatus
5 controller
7 inserted-portion main unit
7a through-hole
7b notch
8 arm
8a end effector
9 endoscope
11 first flexing joint
12 intermediate roll joint
13 second flexing joint
14,15 pivoting member
16a wire (driving wire rod)
22 base-end-side roll joint
23 guide groove
23a opening part

## Claims

1. A treatment manipulator (2) comprising:
an inserted-portion main unit (7);
at least an arm (8) that is provided so as to protrude forward from a distal-end surface of the inserted-portion main unit (7) and that has an end effector (8a) at a distal end thereof; and
an endoscope (9) that is provided in the inserted-portion main unit (7) and that has a viewing-field area (R) in which the end effector (8a) at the distal end of the arm (8) can be observed,
wherein the arm (8) is provided with, sequentially from the distal-end side, a first flexing joint (11) that can pivot the end effector (8a) about a first axis (A2) orthogonal to a longitudinal axis (A1) of the arm (8), an intermediate roll joint (12) that can be rotated about the longitudinal axis (A1), and a second flexing joint (13) that can be pivoted about a second axis (A3) orthogonal to the longitudinal axis (A1), wherein the first flexing joint (11) can be flexed 90° or more only on one side with respect to the longitudinal axis (A1),
a driving wire rod (16a) that drives the end effector (8a) is disposed so as to pass through interiors of the individual joints (11, 12, 13) constituting the arm (8), and
the first flexing joint (11) has, at the interior thereof, a pathway (14a, 15a) through which the driving wire rod (16a) passes, and is provided with a pivoting member (14, 15) that can be passively pivoted about a flexing center of the first flexing joint (11) in response to the operation of the bending driving wire rod (16a).

2. A treatment manipulator (2) according to Claim 1,
wherein the inserted-portion main unit (7) is provided with a through-hole (7a) that passes through the inserted-portion main unit (7) in a longitudinal direction so as to open at the distal-end surface thereof and that accommodates the endoscope (9) in a movable manner, and
the through-hole (7a) is provided with a notch (7b) having a larger width than an outer diameter of the endoscope (9) over a predetermined length from an opening at the distal-end surface.

3. A treatment manipulator (2) according to Claims 1 or 2, further comprising:
a base-end-side roll joint (22) that is provided closer to the base-end side than the second flexing joint (13) is so as to be rotatable about the longitudinal axis (A1).

4. A treatment manipulator (2) according to any one of Claims 1 to 3, further comprising:
a guide groove (23) that is provided in an outer surface of the inserted-portion main unit (7) along the longitudinal direction thereof, that has an opening part (23a) that opens radially outward, and into which a tubular guide member can be inserted from a radially outward direction via the opening part (23a).

5. A manipulator system (1) comprising:
a slave apparatus (3) provided with a treatment manipulator (2) according to any one of Claims 1 to 4 and a driving portion that drives the treatment manipulator (2);
a master apparatus (4) provided with a manipulation portion that is manipulated by an operator (A); and
a controller (5) that controls the driving portion of the slave apparatus (3) based on an input signal input via the manipulation portion of the master apparatus (4).

6. A manipulator system (1) according to Claim 5, wherein, when generating operation instruction signals for the individual joints (11, 12, 13) of the slave apparatus (3) based on input signals received from the master apparatus (4), the controller (5) calculates the operation instruction signals for the individual joints (11, 12, 13) so that the first flexing joint (11) is disposed at a position further away from a center of an endoscope image.

## Patentansprüche

1. Behandlungsmanipulator (2), umfassend:
eine Einführabschnitt-Haupteinheit (7);
mindestens einen Arm (8), der dazu vorgesehen ist, von einer Distalendfläche der Einführabschnitt-Haupteinheit (7) nach vorn vorzustehen, und der einen Endeffektor (8a) an einem distalen Ende davon aufweist; und
ein Endoskop (9), das in der Einführabschnitt-Haupteinheit (7) vorgesehen ist und eine Sichtfeldfläche (R) aufweist, in der der Endeffektor (8a) an dem distalen Ende des Arms (8) beobachtet werden kann,
wobei der Arm (8), von der Distalendseite aufeinanderfolgend, mit einem ersten Biegegelenk (11), das den Endeffektor (8a) um eine erste Achse (A2) orthogonal zu einer Längsachse (A1) des Arms (8) schwenken kann, einem zwischenliegenden Rollgelenk (12), das um die Längsachse (A1) gedreht werden kann, und einem zweiten Biegegelenk (13), das um eine zweite Achse (A3) orthogonal zu der Längsachse (A1) geschwenkt werden kann, versehen ist,
wobei das erste Biegegelenk (11) an nur einer Seite mit Bezug auf die Längsachse (A1) um 90° oder mehr gebogen werden kann,
eine Antriebsdrahtstange (16a), die den Endeffektor (8a) antreibt, derart angeordnet ist, dass sie durch das Innere der einzelnen Gelenke (11, 12, 13), die den Arm (8) bilden, durchgeht, und
das erste Biegegelenk (11) im Inneren davon eine Bahn (14a, 15a) aufweist, durch die die Antriebsdrahtstange (16a) durchgeht, und mit einem Schwenkelement (14, 15) versehen ist, das in Reaktion auf den Vorgang des Biegens der Antriebsdrahtstange (16a) passiv um einen Biegemittelpunkt des ersten Biegegelenks (11) geschwenkt werden kann.

2. Behandlungsmanipulator (2) nach Anspruch 1,
wobei die Einführabschnitt-Haupteinheit (7) mit einem Durchgangsloch (7a) versehen ist, das in einer Längsrichtung durch die Einführabschnitt-Haupteinheit (7) durchgeht, um sich an der Distalendfläche davon zu öffnen, und das Endoskop (9) auf bewegliche Weise aufnimmt, und
das Durchgangsloch (7a) mit einer Kerbe (7b) versehen ist, die eine größere Breite als ein Außendurchmesser des Endoskops (9) über eine vorbestimmte Länge einer Öffnung an der Distalendfläche aufweist.

3. Behandlungsmanipulator (2) nach Anspruch 1 oder 2, ferner umfassend:
ein Basisendseitenrollgelenk (22), das näher an der Basisendseite vorgesehen ist als das zweite Biegegelenk (13), um um die Längsachse (A1) drehbar zu sein.

4. Behandlungsmanipulator (2) nach einem der Ansprüche 1 bis 3, ferner umfassend:
eine Führungsnut (23), die in einer Außenfläche der Einführabschnitt-Haupteinheit (7) entlang der Längsrichtung davon vorgesehen ist, die einen Öffnungsteil (23a) aufweist, der sich radial nach außen öffnet, und in den von einer radialen Außenrichtung über den Öffnungsteil (23a) ein schlauchförmiges Führungselement eingeführt werden kann.

5. Manipulatorsystem (1), umfassend:
eine Slave-Vorrichtung (3), die mit einem Behandlungsmanipulator (2) nach einem der Ansprüche 1 bis 4 und einem Antriebsabschnitt versehen ist, der den Behandlungsmanipulator (2) antreibt;
eine Master-Vorrichtung (4), die mit einem Manipulationsabschnitt versehen ist, der von einem Bediener (A) manipuliert wird; und
eine Steuereinrichtung (5), die den Antriebsabschnitt der Slave-Vorrichtung (3) basierend auf einem Eingabesignal steuert, das über den Manipulationsabschnitt der Master-Vorrichtung (4) eingegeben wurde.

6. Manipulatorsystem (1) nach Anspruch 5, wobei, beim Erzeugen von Bedienanweisungssignalen für die einzelnen Gelenke (11, 12, 13) der Slave-Vorrichtung (3) basierend auf Eingabesignalen, die von der Master-Vorrichtung (4) empfangen wurden, die Steuereinrichtung (5) die Bedienanweisungssignale für die einzelnen Gelenke (11, 12, 13) derart berechnet, dass das erste Biegegelenk (11) an einer Position angeordnet ist, die weiter von einem Mittelpunkt eines Endoskopbilds entfernt ist.

## Revendications

1. Manipulateur de traitement (2) comprenant :
une unité principale de partie insérée (7) ;
au moins un bras (8) qui est disposé de façon à faire saillie vers l'avant à partir d'une surface d'extrémité distale de l'unité principale de partie insérée (7) et qui a un effecteur terminal (8a) à une extrémité distale de celui-ci ; et
un endoscope (9) qui est disposé dans l'unité principale de partie insérée (7) et qui a une zone de champ de vision (R) dans laquelle l'effecteur terminal (8a) à l'extrémité distale du bras (8) peut être observé,
le bras (8) comportant, séquentiellement à partir du côté extrémité distale, une première articulation de flexion (11) qui peut faire pivoter l'effecteur terminal (8a) autour d'un premier axe (A2) orthogonal à un axe longitudinal (A1) du bras (8), une articulation de roulis intermédiaire (12) qui peut être amenée à tourner autour de l'axe longitudinal (A1), et une seconde articulation de flexion (13) qui peut être amenée à pivoter autour d'un second axe (A3) orthogonal à l'axe longitudinal (A1),
la première articulation de flexion (11) pouvant être fléchie à 90° ou plus uniquement sur un côté par rapport à l'axe longitudinal (A1),
une tige de fil d'entraînement (16a) qui entraîne l'effecteur terminal (8a) étant disposée de façon à passer à travers les intérieurs des articulations individuelles (11, 12, 13) constituant le bras (8), et
la première articulation de flexion (11) ayant, à l'intérieur de celle-ci, un passage (14a, 15a) à travers lequel passe la tige de fil d'entraînement (16a), et comportant un élément de pivotement (14, 15) qui peut être amené à pivoter de manière passive autour d'un centre de flexion de la première articulation de flexion (11) en réponse à l'actionnement de la tige de fil d'entraînement de courbure (16a).

2. Manipulateur de traitement (2) selon la revendication 1,
dans lequel l'unité principale de partie insérée (7) comporte un trou traversant (7a) qui passe à travers l'unité principale de partie insérée (7) dans une direction longitudinale de façon à déboucher à la surface d'extrémité distale de celle-ci et qui reçoit l'endoscope (9) d'une manière mobile, et
le trou traversant (7a) comportant une encoche (7b) ayant une largeur plus grande qu'un diamètre externe de l'endoscope (9) sur une longueur prédéterminée à partir d'une ouverture à la surface d'extrémité distale.

3. Manipulateur de traitement (2) selon la revendication 1 ou 2, comprenant en outre :
une articulation de roulis côté extrémité de base (2) qui est disposée plus proche du côté extrémité de base que la seconde articulation de flexion (13) de façon à être apte à tourner autour de l'axe longitudinal (A1).

4. Manipulateur de traitement (2) selon l'une quelconque des revendications 1 à 3, comprenant en outre :
une rainure de guidage (23) qui est disposée dans une surface externe de l'unité principale de partie insérée (7) le long de la direction longitudinale de celle-ci, qui a une partie d'ouverture (23a) qui s'ouvre radialement vers l'extérieur, et dans laquelle un élément de guidage tubulaire peut être inséré depuis une direction radialement vers l'extérieur par l'intermédiaire de la partie d'ouverture (23a).

5. Système de manipulateur (1) comprenant :
un appareil esclave (3) comportant un manipulateur de traitement (2) selon l'une quelconque des revendications 1 à 4 et une partie d'entraînement qui entraîne le manipulateur de traitement (2) ;
un appareil maître (4) comportant une partie de manipulation qui est manipulée par un opérateur (A) ; et
une unité de commande (5) qui commande la partie d'entraînement de l'appareil esclave (3) sur la base d'un signal d'entrée entré par l'intermédiaire de la partie de manipulation de l'appareil maître (4).

6. Système de manipulateur (1) selon la revendication 5, dans lequel, lors de la génération de signaux d'instruction d'actionnement pour les articulations individuelles (11, 12, 13) de l'appareil esclave (3) sur la base de signaux d'entrée reçus en provenance de l'appareil maître (4), l'unité de commande (5) calcule les signaux d'instruction d'actionnement pour les articulations individuelles (11, 12, 13) de telle sorte que la première articulation de flexion (11) est disposée dans une position davantage éloignée d'un centre d'une image d'endoscope.
